# EUROPÄISCHE PATENTANMELDUNG

(11) **EP 0 550 908 A2**
(43) Veröffentlichungstag der Anmeldung: **14.07.1993**
(21) Anmeldenummer: 92122082.8
(22) Anmeldetag: 28.12.1992
(51) Int. Cl.: C07C 263/20

(54) **Verfahren zur Isolierung von Polyisocyanat-Isomeren aus Polyisocyanatgemischen**

(30) Priorität: 08.01.1992 DE 4200236
(71) Anmelder: BAYER AG, 51368 Leverkusen (DE)
(72) Erfinder: Friedrichs, Wolfgang, Dr., W-5000 Köln 41 (DE)

(57) **Zusammenfassung**

Die Erfindung betrifft ein neues Verfahren zur Isolierung von Polyisocyanat-Isomeren in reiner oder angereicherter Form aus mindestens zwei Polyisocyanat-Isomere enthaltenden Polyisocyanatgemischen durch flüssig-flüssig-Extraktion unter Verwendung eines zweiphasigen Systems bestehend aus einer unpolaren und einer polaren, Halogenwasserstoff enthaltenden Phase.

## Beschreibung

Die Erfindung betrifft ein neues Verfahren zur Isolierung von Polyisocyanat-Isomeren in reiner oder angereicherter Form aus mindestens zwei Polyisocyanat-Isomere enthaltenden Polyisocyanatgemischen durch flüssig-flüssig-Extraktion unter Verwendung eines zweiphasigen Systems bestehend aus einer unpolaren und einer polaren, Halogenwasserstoff enthaltenden Phase.

Es ist bereits in der Literatur beschrieben, daß Polyisocyanatgemische durch fraktionierte Destillation gereinigt werden können, So wird beispielsweise in DE-OS 3 145 010 die Isolierung von hochreinem 4,4'-Diisocyanatodiphenylmethan (im folgenden 4,4'-MDI genannt) aus einer Polyisocyanatmischung, wie sie durch Phosgenierung von Anilin/Formaldehyd-Kondensationsprodukten erhalten wird, beschrieben. Nachteilig bei diesem fünfstufigen Destillationsverfahren sind der hohe apparative Aufwand für die mehrfache Destillation der Polyisocyanatgemische und die damit verbundene hohe thermische Belastung für das Verfahrensprodukt 4,4'-MDI.

In DE-OS 2 425 658 wird die Trennung von 2,4'-Diisocyanatodiphenylmethan (im folgenden 2,4'-MDI genannt) und 4,4'-MDI aus Polyisocyanatgemischen der in DE-OS 3 145 010 beschriebenen Art durch destillative Vorreinigung und anschließende Kristallisation beschrieben, Dabei wird zunächst aus dem rohen Polyisocyanatgemisch ein 2,4'-MDI und 4,4'-MDI enthaltendes Destillat gewonnen, aus dem beim Abkühlen vor, 40°C auf 18°C innerhalb von 5 - 6 Stunden 4,4'-MDI in hoher Reinheit auskristallisiert, Nachteilig bei diesem Verfahren sind die für die Kristallisation nötige lange Abkühlzeit und die damit verbundenen hohen Anforderungen an die Temperaturführung innerhalb des Verfahrensprozesses.

Die kontinuierliche Trennung von Polyisocyanat-Isomerengemischen aus 2,4-Toluylendiisocyanat (im folgenden 2,4-TDI genannt) und 2,6-Toluylendiisocyanat (im folgenden 2,6-TDI genannt) durch partielle Kristallisation in einem Rohrkristallisator wird in DE-OS 3 220 439 beschrieben. Dabei wird das Isomerengemisch beim Durchleiten durch ein gekühltes Rohr von 30°C innerhalb von 5 Stunden auf 6°C abgekühlt. Als Kristallisat wird ein 2,4-TDI-reiches Produkt erhalten, das nach Aufschmelzen auf 20°C und abziehen der sich abscheidenden Mutterlauge weitergereinigt werden kann, Nachteilig bei diesem Verfahren ist die zur Kristallisation nötige lange Zeit.

Nach dem gleichen Prinzep arbeitet US 4 246 187. In einem als Schälschleuder ausgebildeten Wärmetauscher wird das zu trennende Isomerengemisch aus 2,4-TDI und 2,6-TDI auf Temperaturen um 6°C gekühlt, die erhaltene Mutterlauge abzentrifugiert und das Kristallisat kontinuierlich ausgeschleust. Nachteilig bei diesem Verfahren ist die technisch aufwendige kontinuierliche Ausschleusung von Feststoffen aus dem Reaktionsgemisch.

In der Anmeldung JP 49 003 981 werden die Stereoisomere des 4,4'-Diisocyanatodicyclohexylmethans (im folgenden H₁₂-MDI genannt), daß heißt cis,cis-, cis,trans- und trans,trans-Isomer, durch fraktionierte Kristallisation aus aliphatischen Kohlenwasserstoffen getrennt. Dazu wird das Stereoisomerengemisch bei Temperaturen um 40°C vollständig in dem verwendeten Lösungsmittel gelöst und das beim Abkühlen auf 10°C auskristallisierende trans,trans-reiche H₁₂-MDI-Isomerengemisch abgetrennt.

Ein besserer Trenneffekt wird in der Anmeldung JP 53 046 944 zur Abtrennung des reinen trans,trans-Stereoisomeren des H₁₂-MDI's dadurch erzielt, daß das Ausgangs-Isomerengemisch in organischen Lösungsmitteln, beispielsweise o-Chlorbenzol, mit gasförmigem Chlorwasserstoff behandelt wird. Die sich bildenden Carbamidsäurechloride sind schwerer löslich als die freien Isocyanate und fallen aus der Reaktionsmischung aus. Nachteilig bei diesem Verfahren ist der technische Aufwand zur kontinuierlichen Abtrennung der als Feststoffe anfallenden Carbamidsäurechloride aus dem Reaktionsgemisch.

Die Trennung von Isomerengemischen aus 2,4-TDI und 2,6-TDI nach dem oben genannten Verfahren, also durch Einleiten von gasförmigem Halogenwasserstoff in eine benzolische Lösung ist ebenfalls beschrieben (A.S.Shevlyakov u.a., Sin.Fiz.-Khim.Polim., 5, 66 (1968); CA 70, 3390 h).

Die bekannten Verfahren haben das gemeinsame Merkmal, daß die gereinigten Polyisocyanat-Isomeren entweder als Feststoff anfallen, so daß die kontinuierliche Aufarbeitung der Verfahrensprodukte nur mit hohem technischem Aufwand möglich ist oder daß die gereinigten Polyisocyanat-Isomeren durch Destillation gewonnen werden, wobei aufgrund der damit verbundenen thermischen Belastung gegebenenfalls Nebenprodukte erzeugt werden können, die die Verfahrensprodukte verunreinigen können.

Aufgabe war es daher, ein Verfahren zur Verfügung zu stellen, daß es gestattet, Polyisocyanatisomere aus Gemischen schonend und großtechnisch durchführbar zu isolieren, wobei die Verarbeitung von festen Verfahrensprodukten vermieden wird.

Gegenstand der Erfindung ist ein Verfahren zur Isolierung von Polyisocyanat-Isomeren in reiner oder angereicherter Form, gegebenenfalls in Form ihrer Carbamidsäurehalogenide, als Lösungen in aprotischen organischen Lösungsmitteln aus mindestens zwei unterschiedliche Isomere enthaltenden Polyisocyanatgemischen, dadurch gekennzeichnet, daß man in einem ersten Arbeitsgang das Polyisocyanat-Ausgangsgemisch in einem zweiphasigen System, bestehend aus
a) einer unpolaren organischen Lösungsmittelphase die gegebenenfalls Polyisocyanate der in der polaren Lösung in reiner oder angereicherter Form zu gewinnenden Art enthält, und
b) einer polaren Lösung eines wasserfreien, bezogen auf die Gesamtmenge der vorliegneden Polyisocyanate in einer unterstöchiometrischen Mengen vorliegenden Halogenwasserstoffs, die gegebenenfalls Polyisocyanate der in dem unpolaren Lösungsmittel in reiner oder angereicherter Form zu gewinnenden Art enthält,
unter Durchmischung der Phasen verteilt und die hierbei anfallenden Phasen nach ihrer Trennung isoliert, wonach gegebenenfalls in einem zweiten Arbeitsgang die unpolare Lösungsmittelphase erneut mit einer polaren Lösungsmittelphase der obengenannten Art und/oder die polare Lösungsmittelphase mit einer unpolaren Lösungsmittelphase der oberngenannten Art extrahiert wird und anschließend die Phase mit den Polyisocyanat-Isomeren in reiner oder angereicherter Form aufarbeitet.

Bevorzugt wird die Durchmischung und anschließende Trennung der Phasen des ersten Arbeitsgangs in einem ein- oder mehrstufigen Gegenstromextraktor durchgeführt.

Die beim ersten Arbeitsgang erhaltene unpolare Lösungmittelphase wird vorzugsweise einer mehrstufigen Extraktion mit einer polaren Phase der oben genannten Art nach dem Gegenstromprinzip unterworfen.

Die beim ersten Arbeitsgang erhaltene polare Lösungsmittelphase kann ebenfalls bevorzugt einer mehrstufigen Extraktion mit einer unpolaren Phase der oben genannten Art nach dem Gegenstromprinzip unterworfen werden,

Bevorzugt wird mindestens eine der erhaltenen Lösungsmittelphasen destillativ aufgearbeitet und die erhaltenen Polyisocyanatgemische gegebenenfalls durch Tempern bei Temperaturen von 20°C bis 200°C von Halogenwasserstoffen befreit.

Die Polyisocyanate verteilen sich in einem zweiphasigen System zwischen den beiden Phasen, Bei der Anwendung auf Mehrkomponentensysteme (Polyisocyanatgemisch) wird eine Anreicherung einer oder mehrerer Komponenten des Mehrkomponentensystem in einer der beiden Phasen erreicht, Diese Anreicherung findet auch statt, wenn die Extraktion der Polyisocyanate in Abwesenheit von Halogenwasserstoffen durchgeführt wird, Die Anreicherung einer oder mehrerer Polyisocyanat-Isomeren in einer der beiden Phasen läßt sich jedoch effektiver gestalten, wenn dem Mehrkomponentensystem Halogenwasserstoffe zugesetzt werden, Durch die Reaktivitätsunterschiede der verschiedenen Isocyanatgruppen in Polyisocyanat-Isomeren reichern sich die Isomeren mit den reaktiveren Gruppen in der polaren Phase an.

Ausgangsmaterialien für das erfindungsgemäße Verfahren sind rohe Polyisocyanatgemische, wie sie bei der großtechnischen Herstellung, beispielsweise durch Phosgenierung der ihnen zugrundliegenden Polyamine, erhalten werden, Bevorzugt werden als Ausgangsmaterialien solche Polyisocyanatgemische eingesetzt, die mindestens zwei stereo- und/oder stellungsisomere Polyisocyanate enthalten. Besonders bevorzugte Polyisocyanatgemische sind die in der Polyurethanchemie in großen Mengen eingesetzten Isomerengemische des Toluylendiisocyanats, des Diisocyanatodiphenylmethans (gegebenenfalls in Gegenwart weiterer oligomerer Derivate), des 1,4'-Diisocyanatocyclohexans und des 4,4'-Diisocyanatodicyclohexylmethans.

Als polare Lösungsmittel eignen sich im erfindungsgemäßen Verfahren alle Lösungsmittel, die unter den Verfahrensbedingungen
a) flüssig sind,
b) die Verfahrensprodukte ausreichend lösen,
c) gegenüber Isocyanaten, Carbamidsäurehalogeniden und Halogenwasserstoffen inert sind, und
d) mit den weiter unten beschriebenen unpolaren Lösungsmitteln mindestens eine Mischungslücke aufweisen.

Diesen Kriterien entsprechende geeignete Lösungsmittel sind beispielsweise aliphatische Sulfone wie Diethylsulfon, Dipropylsulfon, Dibutylsulfon, Ethylpropylsulfon; cyclische Sulfone wie Sulfolan, 2-Methylsulfolan, 3-Methylsulfolan, 2,4-Dimethylsulfolan; aromatische Nitroverbindungen wie Nitrobenzol, 2-Nitrotoluol, 3-Nitrotoluol, 4-Chlornitrobenzol; aliphatische Nitrile wie Acetonitril; sowie Gemische derartiger Verbindungen. Vorzugsweise werden als polare Lösungsmittel Acetonitril, Sulfolan oder 3-Methylsulfolan eingesetzt.

Als unpolare Lösungsmittel eignen sich im erfindungsgemäßen Verfahren alle Lösungsmittel, die unter den Verfahrensbedingungen
a) flüssig sind,
b) die Verfahrensprodukte ausreichend lösen,
c) gegenüber Isocyanaten, Carbamidsäurehalogeniden und Halogenwasserstoffe inert sind, und
d) mit den oben beschriebenen polaren Lösungsmitteln mindestens eine Mischungslücke aufweisen.

Diesen Kriterien entsprechende geeignete Lösungsmittel sind beispielsweise aliphatische Kohlenwasserstoffe wie n-Hexan, i-Octan, den Bedingungen a) bis d) genügende Benzinfraktionen; cycloaliphatische Kohlenwasserstoffe wie Cyclohexan, Methylcyclohexan; aromatische Kohlenwasserstoffe wie Benzol, Toluol; sowie Gemische derartiger Verbindungen. Vorzugsweise werden als unpolare Lösungsmittel n-Hexan, i-Octan, Cyclohexan oder Methylcyclohexan eingesetzt.

Die zu trennenden Polyisocyanatgemische können wahlweise der polaren, Halogenwasserstoff enthaltenden oder der unpolaren Lösungsmittelphase oder beiden zugesetzt werden. Die Konzentration der Polyisocyanate richtet sich nach deren Löslichkeit und nach der Löslichkeit der entsprechenden Carbamidsäurehalogenide in den verwendeten Lösungsmitteln. In der Regel werden 1 -50 Gew.-%ige Lösungen eingesetzt. Vorzugsweise werden mindestens 5 Gew.-%ige Lösungen eingesetzt, da bei kleineren Konzentrationen nur geringe Raum-Zeit-Ausbeuten realisierbar sind,

Als Halogenwasserstoffe werden Chlorwasserstoff, Bromwasserstoff oder Gemische der genannten Halogenwasserstoffe eingesetzt. Selbstverständlich können auch unter den Verfahrensbedingungen Halogenwasserstoff freisetzende Verbindungen verwendet werden. Als Halogenwasserstoff freisetzende Verbindungen sind von tertiären Aminen abgeleitete, in polaren Lösungsmitteln lösliche Ammonium halogenide, beispielsweise Trimethylammoniumchlorid, Triethylammoniumbromid oder Methyldiphenylammoniumchlorid, oder primäre Carbamidsäurehalogenide, beispielsweise N- Methylcarbamidsäurechlorid, N-Phenylcarbamidsäurechlorid oder vorzugsweise die von den zu trennenden Polyisocyanaten abgeleiteten Carbamidsäurehalogenide geeignet.

Die Volumenverhältnisse zwischen polarer und unpolarer Phase können bei allen Stufen des erfindungsgemäßen Verfahrens, insbesondere in Abhängigkeit von der Zusammensetzung des aufzutrennenden Polyisocyanatgemischs, innerhalb weiter Grenzen, beispielsweise 1:10 bis 10:1, vorzugsweise 1:5 bis 5:1 schwanken.

Die Menge an zugesetztem Halogenwasserstoff wird vorzugsweise bei allen Stufen des erfindungsgemäßen Verfahrens so gewählt, daß in den polaren Phasen bis 1 Mol Halogenwasserstoff pro Mol der in der polaren Phase abzutrennenden Polyisocyanate vorliegen.

Die Art, wie der Halogenwasserstoff in das System eingebracht wird, ist für das erfindungsgemäße Verfahren von untergeordneter Bedeutung. So kann der Halogenwasserstoff beispielsweise in einer der beiden Phasen oder in beiden Phasen gelöst werden Es ist selbstverständlich auch möglich, einen Teil des zu trennenden Polyisocyanatgemisches vor der Extraktion durch Reaktion mit gasförmigem Halogenwasserstoff in die entsprechenden Carbamidsäurehalogenide zu überführen und somit den Halogenwasserstoff in gebundener Form, vorzugsweise in der polaren Lösungsmittelphase, dem System zuzuführen.

Die Bauart der beim erfindungsgemäßen Verfahren eingesetzten Extraktionsapparaturen ist von untergeordneter Bedeutung. Es können die in der chemischen Technologie für entsprechende Trennungsoperationen üblichen Vorrichtungen zum Einsatz gelangen. Beispiele hierfür sind mehrstufig arbeitende Gegenstromextraktionskolonnen oder auch in Serie geschaltete Mischer-Scheider-Batterien. Vorzugsweise wird das erfindungsgemäße Verfahren unter Verwendung von 5- bis 15-stufig arbeitenden Gegenstromextraktionskolonnen durchgeführt.

Bei allen Verfahrensstufen wird im allgemeinen unter solchen Temperatur- und Druckbedingungen gearbeitet: innerhalb derer beide Phasen flüssig sind. Vorzugsweise wird das Verfahren innerhalb des Temperaturbereichs von 10°C bis 150°C unter Normaldruck durchgeführt. Es rann zweckmäßig sein: die einzelnen Verfahrensschritte bei unterschiedlichen Temperaturen durchzuführen. Die Anwendung von Über- oder Unterdruck ist selbstverständlich nicht prinzipiell ausgeschlossen.

Die Aufarbeitung der unpolaren Phase erfolgt bevorzugt durch destillative Abtrennung des Lösungsmittels. Das resultierende Polyisocyanat-Isomerengemisch, das gegebenenfalls geringe Mengen an Carbamidsäurehalogeniden enthält, kann durch Tempern bei Temperaturen, die ausreichen, das Carbamidsäurehalogenid zumindest teilweise in freies Isocyanat und Halogenwasserstoff zu dissoziieren, gegebenenfalls unter Durchleiten eines Inertgasstromes, von Restmengen an Halogenwasserstoff befreit werden. Im allgemeinen erfolgt der Temperungsschritt bei Temperaturen von 20°C bis 200°C. Selbstverständlich kann das Polyisocyanat- Isomerengemisch auch ohne vorherige Temperung weiterverarbeitet werden, wenn es beispielsweise einer Verwendung zugeführt -werden soll, bei der die Anwesenheit von Halogenwasserstoffen nicht stört oder gegebenenfalls sogar erwünscht ist.

Die polare Phase, die im allgemeinen einen Großteil des eingesetzten Halogenwasserstoffs in Form von Carbamidsäurehalogeniden enthält, kann in gleicher Weise aufgearbeitet werden wie die unpolare Phase.

Selbstverständlich kann die unpolare und/oder polare Phase ganz oder teilweise in das Extraktionsverfahren, zurückgeführt werden. So kann es in manchen Fällen vorteilhaft sein, das in der polaren Phase vorliegende Carbamidsäurehalogenid als Halogenwasserstoff freisetzende Verbindung für das erfindungsgemäße Verfahren einzusetzen. Dazu kann die polare Phase ohne weitere Reinigung eingesetzt werden.; die polare Phase kann selbstverständlich auch durch Abdestillieren des Lösungsmittels oder eines Teils des Lösungsmittel vor der Rückführung aufkonzentriert werden.

Desweiterer kann die unpolare und/oder polare Phase selbstverständlich auch in weiteren Verfahrensschritten erneut wie im ersten Verfahrensschritt extrahiert werden, um eine effektivere Isolierung der Polyisocyanat-Isomeren in reiner oder angereicherter Form zu erzielen.

Die nachfolgenden Beispiele dienen zur näheren Erläuterung des erfindungsgemäßan Verfahrens, ohne dieses zu beschränken.

### Beispiel 1

In einer 10-stufigen Mischer-Scheider-Anlage (mixer-settler-Anlage) werden bei 40°C eine Lösung von 385 g eines Toluylendiisocyanatgemischs (Isomerengemisch aus 35 Gew.-% 2,6-Isomer und 65 Gew.-%, 2,4-Isomer) und 80 g Chlorwasserstoff in 440 g wasserfreiem Acetonitril und eine Lösung von 165 g Toluylendiisocyanatgemisch (Isomerengemisch aus 35 Gew.-% 2,6 Isomer und 65 Gew.-% 2,4-Isomer) in 1450 g wasserfreiem Cyclohexan einander entgegengefahren Dabei werden 1000 ml/h der Acetonitrillösung und 2000 g/h der Cyclohexanlösung durchgesetzt. Nach ca. 4 h Einfahrzeit hat sich in der Anlage ein Gleichgewicht eingestellt und als Verfahrensprodukte werden 1770 g/h Acetonitrilphase und 715 g/h Cyclohexanphase erhalten. Die Acetonitrilphase enthält laut Gas chromatographie 285 g/kg Toluylendiisocyanatgemisch (Isomerengemisch aus 33 Gew.-%, 2,6-Isomer und 67 Gew.-% 2,4-Isomer), wobei ein Teil der Toluylendiisocyanate in Form der entsprechenden Carbamidsäurechloride vorliegt. Die Cyclohexanphase enthält 67 g/kg Toluylendiisocyanatgemich (Isomerengemisch aus 59 Gew.-% 2,6-Isomer und 41 Gew.-% 2,4-Isomer).

Aus dem eingesetzten Toluylendiisocyanat-Isomerengemisch (Isomerenverhältnis 35:65) werden also zwei neue Isomerengemische mit den Isomerenverhältnisse 33:67 und 59:41 gewonnen.

### Beispiel 2

Zu einer Losung von 2000 g eines Diisocyanatodiphenylmethangemisches (Isomerengemisch aus 50 Gew.-% 4,4'-Isomer und 50 Gew.-% 2,4'-Isomer) in 8000 g wasserfreiem Cyclohexan gibt man eine Lösung von 265 g Chlorwasserstoff in 2250 g frisch absolutiertem Sulfolan. Nach 1 h intensivem Rühren bei 40°C werden die Phasen getrennt. Man erhält 4450 g Sulfolanphase und 8350 g Cyclohexanphase. Die Sulfolanphase enthält laut Gaschromatographie 1645 g Diisocyanatodiphenylmethangemisch (Isomerengemisch aus 56 Gew.-% 4,4'-Isomer und 44 Gew.-% 2,4'-Isomer), wobei ein Teil der Diisocyanatodiphenylmethane in Form der entsprechenden Carbamidsäurehalogenide vorliegt. Die Cyclohexanphase liefert nach Abziehen des Lösungsmittels in einem Rotationsverdampfer 355 g Diisocyanatodiphenylmethangemisch (Isomerengemisch aus 23 Gew.-% 4,4'-Isomer und 77 Gew.-% 2,4'-Isomer).

Aus dem eingesetzten Diisocyanatodiphenylmethan-Isomerengemisch (Isomerenverhältnis 50:50) werden also zwei neue Isomerengemische mit den Isomerenverhältnissen 56:44 und 23:77 gewonnen.

### Beispiel 3

Zu einer Lösung von 3,0 kg eines 4,4'-Diisocyanatodicyclohexylmethangemisches (Isomerengemisch aus 27 Gew.-% cis,cis-Isomer, 50 Gew,-% cis,trans-Isomer und 23 Gew.-% trans,trans-Isomer) in 9,0 kg wasserfreiem Cyclohexan gibt man eine Lösung von 300 g Chlorwasserstoff in 5,4 kg wasserfreiem Acetonitril. Nach 1 h intensivem Rühren bei 30°C werden die Phasen getrennt. Man erhält 9,8 kg Acetonitrilphase und 7,9 kg Cyclohexanphase. Die Cyclohexanphase liefert nach Abziehen des Lösungsmittels 800 g 4,4'-Diisocyanatodicyclohexylmethan-Gemisch (Isomerengemisch aus 36 Gew.-% cis,cis-Isomer, 50 Gew.-% cis,trans-Isomer und 14 Gew.-% trans-trans-Isomer).

Dieses Gemisch wird mit 2,4 kg wasserfreiem Cyclohexan und einer Lösung von 85 g Chlorwasserstoff in 1,4 kg wasserfreiem Acetonitril versetzt. Nach 1 h intensivem Rühren bei 30°C werden die Phasen getrennt. Man erhält 2,9 kg Acetonitrilphase und 1,8 kg Cyclohexanphase. Die Cyclohexanphase liefert nach Abziehen des Lösungsmittels 135 g 4,4'-Diisocyanatodicyclohexylmethan-Gemisch (Isomerengemisch aus 44 Gew.-% cis,cis-Isomer, 46 Gew.-% cis,trans-Isomer und 10 Gew.-% trans,trans-Isomer).

Aus dem eingesetzten Isomerengemisch (Isomerenverhältnis 27:50:23) wird also ein neues Isomerengemisch mit einem Isomerenverhältnis von 44:46; 10 gewonnen.

## Patentansprüche

1. Verfahren zur Isolierung von Polyisocyanat-Isomeren in reiner oder angereicherter Form, gegebenenfalls in Form ihrer Carbamidsäurehalogenide, als Lösungen in aprotischen organischen Lösungsmitteln aus mindestens zwei unterschiedliche Isomere enthaltenden Polyisocyanatgemischen, dadurch gekennzeichnet, daß man in einem ersten Arbeitsgang das Polyisocyanat-Ausgangsgemisch in einem zweiphasigen System, bestehend aus
a) einer unpolaren organischen Lösungsmittelphase, die gegebenenfalls bereits Polyisocyanate der in der polaren Lösung in reiner oder angereicherter Form zu gewinnenden Art enthält, und
b) einer polaren Lösung eInes wasserfreien, bezogen auf die Gesamtmenge der vorliegenden Polyisocyanate in einer unterstöchiometrischen Menge vorliegenden Halogenwasserstoffs, die gegebenenfalls bereits Polyisocyanate der in dem unpolaren Lösungsmittel in reiner oder angereicherter Form zu gewinnenden Art enthält,
unter Durchmischung der Phasen verteilt und die hierbei anfallenden Phasen nach ihrer Trennung isoliert, wonach gegebenenfalls in einem zweiten Arbeitsgang die unpolare Lösungsmittelphase erneut mit einer polaren Lösungsmittelphase der obengenannten Art und/oder die polare Lösungsmittelphase mit einer unpolaren Lösungsmittelphase der obengenannten Art extrahiert wird und anschließend die Phase mit den Polyisocyanat-Isomeren in reiner oder angereicherter Form aufarbeitet.

2. Verfahren gemäß Anspruch 1, dadurch gekennzeichnet, daß man die Durchmischung und anschließende Trennung der Phasen des ersten Arbeitsganges in einem ein- oder mehrstufigen Gegenstromextraktor durchführt.

3. Verfahren gemäß Anspruch 1 und 2, dadurch gekennzeichnet, daß man die beim ersten Arbeitsgang erhaltene unpolare Lösungsmittelphase einer mehrstufigen Extraktion mit einer polaren Phase der in Anspruch 1 genannten Art nach dem Gegenstromprihzip unterwirft.

4. Verfahren gemäß Anspruch 1 und 2, dadurch gekennzeichnet, daß man die beim ersten Arbeitsgang erhaltene polare Lösungsmittelphase einer mehrstufigen Extraktion mit einer unpolaren Phase der in Anspruch 1 genannten Art nach dem Gegenstromprinzip unterwirft.

5. Verfahren gemäß Anspruch 1 bis 3, dadurch gekennzeichnet, daß man als Polyisocyanatgemisch solche Isomerengemische einsetzt, die mindestens zwei stereo- und/oder stellungsisomere Polyisocyanate enthalten.

6. Verfahren gemäß Anspruch 1 bis 4, dadurch gekennzeichnet, daß man als Polyisocyanatgemische Isomerengemische des Toluylendiisocyanates, Diisocyanatodiphenylmethans, 1,4-Diisocyanatocyclohexans oder 4,4'-Diisocyanatodicyclohexylmethans einsetzt.

7. Verfahren gemäß Anspruch 1 bis 4, dadurch gekennzeichnet, daß man mindestens eine der erhaltenen Lösungsmittelphasen destillativ aufarbeitet und die erhaltenen Polyisocyanatgemische gegebenenfalls durch Tempern bei Temperaturen von 20°C bis 200°C von Halogenwasserstoffen befreit.
